# EUROPEAN PATENT SPECIFICATION

(11) **EP 2 424 493 B1**
(45) Date of publication and mention of the grant of the patent: **25.09.2013**
(21) Application number: 10714652.4
(22) Date of filing: 26.03.2010
(51) Int. Cl.: A61K 9/00, A61K 47/36

(54) **INTIMATE LUBRICATION KIT**
INTIM-GLEITMITTELKIT
KIT DE LUBRIFICATION INTIME

(30) Priority: 01.04.2009 GB 0905677
(43) Date of publication of application: 07.03.2012
(73) Proprietor: Yes Syzygy Limited, Hampshire GU34 3NS (GB)
(72) Inventor: LENNOX, Susan Mary, Hampshire GU34 3NS (GB); BROOKS, Sarah Annabelle, Hampshire GU32 2DJ (GB)
(74) Representative: Brooks, Nigel Samuel
(86) International application number: PCT/GB2010/000601
(87) International publication number: WO 2010/112829

(56) References cited:
- WO-A1-2009/066102
- US-A1- 2006 204 557
- TSUSHIMA KYOGO: "Water-soluble vaginal lubricants" CAPLUS, 26 January 1999 (1999-01-26), XP002385642

## Description

The present invention relates to an intimate lubrication kit.

Couples can experience discomfort when making love. Often this is caused by vaginal dryness.

In International Patent Application No. WO 2006/092581 there is described and claimed - at least on entry into the UK regional phase No GB 2,437,903 A - A lubricating composition comprising:
- linseed extract and a further seed polysaccharide extract soluble in water,
- a pH buffer and
- at least one preservative for prevention of growth of micro-organisms.

In production, this composition has been acidic. Vaginal fluids are acid typically in the region of a pH of 4 to 5. However sperm can be incapacitated by acidity, in that they cease to swim, i.e. become immotile. Sperm can also lose viability if in an aqueous environment where the osmolality is either too high, hyperosmotic (greater than 380 mosm) or too low hypo-osmotic (less than 280 mosm). Semen normally has a pH between 7 & 8 and has an osmolality in the region of 320 mosm, ranging from 260 to 365 mosm.

Vaginal acidity is advantageous in discouraging infections such as thrush (Candida albicans), Bacterial Vaginosis and sexually transmitted infections such as Trichomonas. These infections reduce ability to conceive.

There is a correlation between vaginal dryness and infertility.

US 2006/204557 in the name of Gupta teaches a water-based personal moisturiser and lubricant. This is a sperm friendly lubricant and therefore not optimised for maintaining vaginal health. JP 11021231 in the name of Okamoto Co Ltd teaches a water-based vaginal lubricant comprising various natural products. There is no teaching of the pH of this lubricant. WO 2009066102 in the name of Burd teaches a vaginal lubricant including hyaluronic acid. None of this prior art addresses the contradiction that sperm require a higher pH for survival and motility and yet the vagina requires a lower pH to discourage infection.

We have sought to develop a lubricating composition to help men and women, who are trying to conceive, whether or not they are suffering from infertility. However the fundamental dichotomy of vaginal acidity not suiting the sensibilities of sperm remains.

In seeking to overcome the dichotomy, we have devised and invented a dual composition lubricant, one composition being sperm friendly and the other composition being vagina friendly.

The object of the present invention is to provide a lubrication kit including such compositions.

According to the invention there is provided an intimate lubrication kit comprising:
- at least one first container containing an aqueous lubricating composition, which composition:
   - provides lubricity;
   - is slightly acid or substantially neutral or slightly alkaline and
   - is substantially iso-osmotic with semen and
- at least one second container containing an aqueous moisturising composition, which composition:
   - provides moisturisation,
   - provides lubricity and
   - is slightly to moderately acidic.

By providing "lubricity" is meant providing lubrication in the manner that soapy water lubricates wet hands rubbed together which can then slip easily across each other.

By providing "moisturisation" is meant ability to transport moisture to human tissue.

Preferably the moisturising composition is substantially iso-osmotic with typical vaginal fluids and preferably has a lower pH than the lubricating composition.

It should be particularly noted that both the lubricating and the moisturising compositions can have both lubricating and moisturising properties. The terms "lubricating composition" and " moisturising composition" are used in accordance with the properties that they are primarily intended to contribute during their respective uses. The lubricating composition and the moisturising composition contain substantially identical lubricity and moisturisation providing constituents. Alternatively the lubricating composition may have a comparatively lower viscosity, to enhance its lubricating properties with respect to the moisturising composition and the moisturising composition can have a comparatively lower osmolality, to enhance its moisturising properties with respect to the lubricating composition.

Whilst we can envisage that either or both of the compositions could be formulated with synthetic materials, such as hydroxyethylcellulose, we prefer to formulate them with naturally derived materials, i.e. materials which have not been chemically synthesised, at least as far as possible. Not least, we prefer to use organically derived materials, i.e. materials derived from plants and preferably plants not treated with synthetic chemicals, because they are less likely in our experience to contain impurities such as traces of solvents and catalysts used in their product. For this reason, we further prefer to use organically derived materials that have not been extracted utilising synthetic materials such as extraction solvents, for instance hexane. For instance where we use aloe vera, we prefer to use it dried as opposed to extracted. In particular we prefer to formulate the lubricating composition with either or both of aqueous extract of linseed (flax) and a further seed polysaccharide.

Normally we would expect the compositions to be hydrophilic; however, for lubricity, we can envisage lubricating composition to incorporate emulsified organically derived hydrophobic constituents, namely beeswax, plant oils and butters. Nevertheless we prefer to avoid linseed oil as such on account of its odour on oxidisation.

By careful choice of the water source from which the compositions are prepared, it is expected that the lubricating composition at least will require little or no addition of acid or alkali to bring it to be slightly acid or substantially neutral or slightly alkaline. Similarly, the moisturising composition be mildly acidic without addition. If this cannot be achieved by use of hard or soft water, we prefer to use citric acid, lactic acid or hydrochloric acid if the respective composition is too alkaline or sodium or potassium hydroxide if too acid. Other common acids and alkalis are possible. To maintain neutrality and slight acidity, we prefer to incorporate pH buffers in the composition, such as phosphates.

Again by choice of the water and other substances, the compositions are expected to be substantially iso-osmotic to semen and the vaginal fluids. If the osmolality requires to be adjusted, this may be achieved by use of an organic solute such as a sugar or an inorganic salt such as sodium chloride.

Preferably the lubricating composition will have a pH of 5.5 to 9.0 - more likely 7.0 to 8.5 - and an osmolality of 200 to 400 mosm, typically 280-380 mosm. The pH is intended to be more sperm friendly than vaginal acidity in order to enhance sperm survival and availability for fertilisation, in particular at the fertile period. Similarly the osmolality is intended to be sperm friendly. Further we believe that a target viscosity for this composition is 10,000 to 15,000 centipoise, in order to facilitate love making even with vaginal dryness. However, in so far as nonNewtonian properties are advantageous, be they shear thinning and/or pseudoplasticity and/or thixotropic, we expect that viscosity outside this range will be suitable on occasion.

Preferably the moisturising composition will have a pH of 3.0 to 5.5 - more likely 3.0 to 4.5 - and an osmolality of 100 to 400 mosm, typically 250-310 mosm. The pH is intended to restore temporary deviation from natural vaginal pH, the deviation being in the interests of sperm friendliness. The osmolality is typically lower than that of the lubricating composition for moisturising. Further we believe that a target viscosity for this composition is 12,000 to 30,000 centipoise. In other words not only will the moisturising composition be more acidic to the first's slight acidity / neutrality / slight alkalinity, and we expect it to have a marginally lower osmolality and slightly higher viscosity than the lubricating composition. Nevertheless, we aim for the moisturising composition to be a compromise in being a moisturiser and a lubricant for love making after ovulation at any time until the next fertile period.

We envisage that we can include lactobacillus in the moisturising composition to enhance it's natural occurrence in the vagina.

Additionally we prefer to incorporate a muco-adherent in the moisturising composition. It is possible to incorporate one in the lubricating composition, but this property is preferably enhanced in the moisturising composition, in the interest of moisturisation.

Whilst the compositions can be provided in bottles, preferably the kit comprises a plurality of small containers of the lubricating composition and a few containers of the moisturising composition. More containers of the moisturising composition can be provided for promoting vaginal health and combating vaginal dryness. Preferably the containers are long necked single use containers, typically 40mm to 60mm.

The containers typically contain 3ml to 7ml.

Preferably the kit may also include ovulation strips for determining the timing of ovulation. The kit may also include instructions for use of the lubricating composition for love making during days up to and including ovulation and of the moisturising composition during love making and/or as a moisturiser used after ovulation at any time until the next fertile period.

To help understanding of the invention, a specific embodiment thereof will now be described by way of example and with reference to the accompanying drawings, in which:

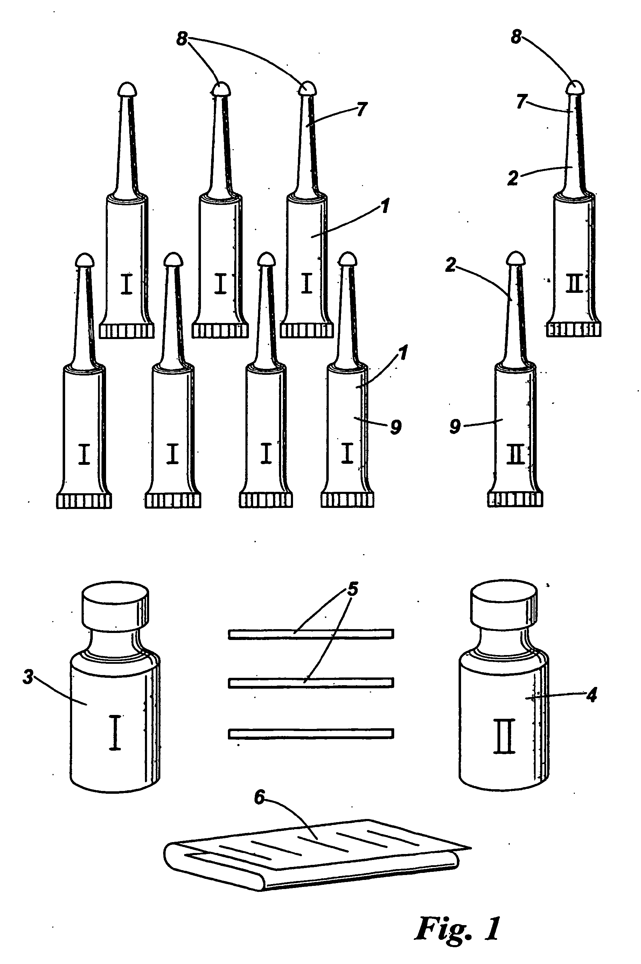

Figure 1 is a diagram of an intimate lubrication kit in accordance with the invention.

Referring to the drawing, the intimate lubrication kit there shown has seven applicators 1 filled with lubricant and three applicators 2 filled with moisturiser. In addition, a bottle 3 of the lubricant and a bottle 4 of the moisturiser are provided. The kit also includes ovulation test strips 5 and instructions 6.

The applicators 1, 2 have long thin necks 7 with closure caps 8 and integral squeezable containers 9. The necks are typically 50mm long and the containers typically have a 5ml capacity. The bottles have 25ml capacity.

Both the lubricant and the moisturiser are prepared in like manner to that described in International Patent Application No. WO 2006/092581 from a common aqueous mixture of linseed extract and Xanthan gum, with small quantities of lonicera caprifolium extract in the lubricant and potassium sorbate in the moisturiser as preservatives.

The lubricant has citric acid or sodium hydroxide added to adjust its pH to 7.0 to 8.5. Further sodium chloride is added to adjust its osmolality to 280-380 mosm. The moisturiser similarly has citric acid added to adjust its pH to 3.0 to 4.5 and its osmolality is adjusted to 250-310 mosm. Further it has aloe vera extract added for its tissue benefits.

We believe these two compositions, i.e. the lubricant and the moisturiser, to be respectively sperm and vagina friendly.

The instructions for use are as follows:

### Intimate Lubrication Kit

*This kit is for couples experiencing difficulty in making love, in particular because of vaginal dryness. It may assist in conception.*

*The kit comprises 7 applicators of lubricating composition for use during the days up to and including ovulation and 3 applicators of a slightly thicker composition for use just after ovulation,*

*The lubricating composition is neutral or slightly acid with a view to being sperm friendly. The thicker composition is slightly acid to mirror vaginal acidity and includes a moisturiser for vaginal friendliness. It is also a lubricant.*

*The lubricating composition is applied to a couple's intimate organs when making love during the woman's most fertile period, i.e. typically the six days up to and including ovulation. Conveniently the composition can be applied to her vagina using the long necked applicators. The moisturising composition is applied to her vagina at least 24hrs after ovulation is expected to have occurred.*

*Ovulation test strips are provided for assistance in determining the timing of ovulation.*

*Some couples may find it more comfortable to apply additional lubricant externally, either from one of the long necked applicators or from a separately provided bottle. Similarly, the moisturiser may be applied externally from the second long necked container or the bottle of moisturiser.*

The invention is not intended to be restricted to the details of the above-described embodiment. For instance, other aqueous polysaccharide aqueous extracts, for instance, Yellow Mustard seed gum, Acacia gum, gums from seaweeds (eg Alginates or Carrageenan), β-Glucans (derived from barley, oats, rye and wheat) and fruit gums. Preferred additional gums are Galactomannan gums, since they exhibit a synergistic phenomena resulting from their Galactomannan structures of long smooth sections of their molecules, for example Locust Bean gum, Guar gum, Cassia gum, Tara Gum, and Konjac Mannan gel which is a Glucomannan and is stereochemically similar to a Galactomannan. Certain of these gums are prepared in the manner of the linseed extract, for instance Yellow Mustard seed gum. Xanthan gum is prepared by action of the Xanthomonas campestris on maize seed. Further in place of potassium sorbate, Citricidal and/or phenoxyethanol may be used as preservatives

Whilst we prefer to use naturally occurring substances, we can envisage use of synthetic polysaccharides, hydroxyethyl cellulose, glycerine, polyethylene oxide, polycarbophil, carbomers and / or glycols.

## Claims

1. An intimate lubrication kit comprising:
• at least one first container containing an aqueous lubricating composition, which composition:
• provides lubricity;
• is slightly acid or substantially neutral or slightly alkaline and
• is substantially iso-osmotic with semen and
• at least one second container containing an aqueous moisturising composition, which composition:
• provides moisturisation,
• provides lubricity and
• is slightly to moderately acidic.

2. An intimate lubrication kit as claimed in claim 1, wherein the moisturising composition is substantially iso-osmotic with typical vaginal fluids and preferably has a lower pH than the lubricating composition.

3. An intimate lubrication kit as claimed in claim 1, or claim 2, wherein either the lubricating composition and the moisturising composition contain substantially identical lubricity and moisturisation providing constituents or the lubricating composition has a comparatively lower viscosity, to enhance its lubricating properties with respect to the moisturising composition and the moisturising composition a comparatively lower osmolality, to enhance its moisturising properties with respect to the lubricating composition.

4. An intimate lubrication kit as claimed in any preceding claim, wherein the compositions are formulated at least substantially with organically derived constituents and preferably at least some of the organically derived constituents have been extracted without utilising synthetic materials and preferably at least one of the organically derived constituents has been dried.

5. An intimate lubrication kit as claimed in any preceding claim, wherein the compositions contain either or both of an aqueous extract of linseed (flax) and a further seed polysaccharide.

6. An intimate lubrication kit as claimed in any preceding claim, wherein the compositions are hydrophobic and preferably the lubricating composition contains emulsified organically derived hydrophobic constituents.

7. An intimate lubrication kit as claimed in any preceding claim, wherein either the pH of the compositions derives from water used in their preparation or the pH of the compositions is controlled by addition of selected ones of citric acid, lactic acid, hydrochloric acid, sodium hydroxide and potassium hydroxide and preferably the compositions contain pH buffers.

8. An intimate lubrication kit as claimed in any preceding claim, wherein the compositions contain an organic solute or an inorganic salt for control of osmolality.

9. An intimate lubrication kit as claimed in any preceding claim, wherein the lubricating composition has a pH of 5.5 to 9.0, and preferably a pH of 7.0 to 8.5, and an osmolality of 200 to 400 mosm and preferably an osmolality of 280-380 mosm and preferably has a viscosity of 10,000 to 15,000 centipoise and preferably with non-Newtonian properties.

10. An intimate lubrication kit as claimed in any preceding claim, wherein the moisturising composition has a pH of 3.0 to 5.5, and preferably a pH of 3.0 to 4.5, and an osmolality of 100 to 400 mosm mosm and preferably an osmolality of 250-310 mosm and preferably has a viscosity of 12,000 to 30,000 centipoise and preferably with non-Newtonian properties.

11. An intimate lubrication kit as claimed in any preceding claim, wherein the moisturising composition contains a muco-adherent or more thereof than the first composition and preferably contains lactobacillus.

12. An intimate lubrication kit as claimed in any preceding claim, wherein the lubricating composition contains lonicera caprifolium and/or the moisturising composition contains potassium sorbate as preservatives.

13. An intimate lubrication kit as claimed in any preceding claim, including a plurality of small containers of the lubricating composition and plurality of containers of the moisturising composition, the containers preferably being long necked single use containers and preferably containing between 3ml and 7ml of their compositions.

14. An intimate lubrication kit as claimed in any preceding claim, including ovulation test strips for determining the timing of ovulation.

15. An intimate lubrication kit as claimed in any preceding claim, including instructions for use of the lubricating composition for love making during the days up to and including ovulation and of the moisturising composition during love making and/or as a moisturiser used after ovulation at any time until the next fertile period.

## Patentansprüche

1. Intim-Gleitmittelkit, das Folgendes umfasst:
• mindestens ein erstes Behältnis, in dem sich ein wässriges Gleitmittel befindet, das
• Gleitfähigkeit bereitstellt,
• leicht sauer oder im Wesentlichen neutral oder leicht alkalisch ist und
• im Wesentlichen isoosmotisch zu Sperma ist, und
• mindestens ein zweites Behältnis, in dem sich ein wässriges Feuchtigkeitsmittel befindet, das
• Feuchtigkeit spendet,
• Gleitfähigkeit bereitstellt und
• leicht bis mäßig sauer ist.

2. Intim-Gleitmittelkit nach Anspruch 1, **dadurch gekennzeichnet, dass** das Feuchtigkeitsmittel im Wesentlichen isoosmotisch zu typischen Vaginalflüssigkeiten ist und vorzugsweise einen niedrigeren pH-Wert als das Gleitmittel besitzt.

3. Intim-Gleitmittelkit nach Anspruch 1 oder Anspruch 2, **dadurch gekennzeichnet, dass** entweder das Gleitmittel und das Feuchtigkeitsmittel im Wesentlichen identische, die Gleitfähigkeit und die Feuchtigkeit fördernde Bestandteile enthalten oder das Gleitmittel eine vergleichsweise geringere Viskosität aufweist, wodurch seine Gleitfähigkeit gegenüber dem Feuchtigkeitsmittel verbessert wird, und das Feuchtigkeitsmittel eine vergleichsweise geringere Osmolalität aufweist, wodurch seine Feuchtigkeit gegenüber dem Gleitmittel erhöht wird.

4. Intim-Gleitmittelkit nach einem der vorhergehenden Ansprüche, **dadurch gekennzeichnet, dass** die Mittel mindestens im Wesentlichen aus Bestandteilen organischer Herkunft hergestellt werden und vorzugsweise mindestens einige der Bestandteile organischer Herkunft ohne Einsatz synthetischer Stoffe extrahiert werden und vorzugsweise mindestens einer der Bestandteile organischer Herkunft getrocknet wird.

5. Intim-Gleitmittelkit nach einem der vorhergehenden Ansprüche, **dadurch gekennzeichnet, dass** die Mittel einen wässrigen Auszug aus Leinsamen (Flachs) und/oder ein Polysaccharid eines weiteren Samens enthalten.

6. Intim-Gleitmittelkit nach einem der vorhergehenden Ansprüche, **dadurch gekennzeichnet, dass** die Mittel hydrophob sind und das Gleitmittel vorzugsweise emulgierte hydrophobe Bestandteile organischer Herkunft enthält.

7. Intim-Gleitmittelkit nach einem der vorhergehenden Ansprüche, **dadurch gekennzeichnet, dass** der pH-Wert der Mittel entweder vom für ihre Herstellung verwendeten Wasser bestimmt oder durch die wahlweise Zugabe von entweder Zitronensäure, Milchsäure, Chlorwasserstoffsäure, Natriumhydroxid und Kaliumhydroxid eingestellt wird und die Mittel vorzugsweise pH-Puffer enthalten.

8. Intim-Gleitmittelkit nach einem der vorhergehenden Ansprüche, **dadurch gekennzeichnet, dass** die Mittel eine organische Beimengung oder ein anorganisches Salz zur Einstellung der Osmolalität enthalten.

9. Intim-Gleitmittelkit nach einem der vorhergehenden Ansprüche, **dadurch gekennzeichnet, dass** das Gleitmittel einen pH-Wert von 5,5 bis 9,0, vorzugsweise einen pH-Wert von 7,0 bis 8,5, eine Osmolalität von 200 bis 400 mosm, vorzugsweise eine Osmolalität von 280 bis 380 mosm, und vorzugsweise eine Viskosität von 10.000 bis 15.000 Centipoise besitzt und vorzugsweise nicht-newtonsche Eigenschaften aufweist.

10. Intim-Gleitmittelkit nach einem der vorhergehenden Ansprüche, **dadurch gekennzeichnet, dass** das Feuchtigkeitsmittel einen pH-Wert von 3,0 bis 5,5, vorzugsweise einen pH-Wert von 3,0 bis 4,5, eine Osmolalität von 100 bis 400 mosm, vorzugsweise eine Osmolalität von 250 bis 310 mosm, und vorzugsweise eine Viskosität von 12.000 bis 30.000 Centipoise besitzt und vorzugsweise nicht-newtonsche Eigenschaften aufweist.

11. Intim-Gleitmittelkit nach einem der vorhergehenden Ansprüche, **dadurch gekennzeichnet, dass** das Feuchtigkeitsmittel im Gegensatz zum erstgenannten Mittel eine oder mehrere an der Schleimhaut anhaftende Substanzen (Mucoadherent) und vorzugsweise Lactobacillus enthält.

12. Intim-Gleitmittelkit nach einem der vorhergehenden Ansprüche, **dadurch gekennzeichnet, dass** das Gleitmittel Lonicera caprifolium und/oder das Feuchtigkeitsmittel Kaliumsorbat als Konservierungsstoff enthält.

13. Intim-Gleitmittelkit nach einem der vorhergehenden Ansprüche, welches mehrere kleine Behältnisse mit Gleitmittel und mehrere Behältnisse mit Feuchtigkeitsmittel umfasst, wobei die Behältnisse vorzugsweise langhalsig und für die einmalige Verwendung bestimmt sind und vorzugsweise zwischen 3 ml und 7 ml des jeweiligen Mittels enthalten.

14. Intim-Gleitmittelkit nach einem der vorhergehenden Ansprüche, welches Ovulations-Teststreifen zur Bestimmung des Ovulationszeitpunkts enthält.

15. Intim-Gleitmittelkit nach einem der vorhergehenden Ansprüche, das Hinweise zur Verwendung des Gleitmittels beim Geschlechtsverkehr an den Tagen bis einschließlich des Eisprungs und des Feuchtigkeitsmittels beim Geschlechtsverkehr und/oder als ein Feuchtigkeitsspender nach dem Eisprung zu einem beliebigen Zeitpunkt bis zur nächsten fruchtbaren Periode enthält.

## Revendications

1. Un nécessaire de lubrification intime comprenant :
• au moins un premier récipient contenant une composition lubrifiante aqueuse, composition qui :
• procure une lubrification ;
• est légèrement acide ou substantiellement neutre ou légèrement alcaline et
• est substantiellement iso-osmotique avec le sperme et
• au moins un second récipient contenant une composition hydratante aqueuse, composition qui :
• procure une hydratation,
• procure une lubrification et
• est légèrement à modérément acide.

2. Un nécessaire de lubrification intime selon la revendication 1, dans lequel la composition hydratante est substantiellement iso-osmotique avec les fluides vaginaux typiques et présente de préférence un pH inférieur à celui de la composition lubrifiante.

3. Un nécessaire de lubrification intime selon la revendication 1, ou la revendication 2, dans lequel soit la composition lubrifiante et la composition hydratante contiennent des constituants substantiellement identiques procurant la lubrification et l'hydratation, soit la composition lubrifiante présente une viscosité comparativement inférieure, pour renforcer ses propriétés lubrifiantes par rapport à la composition hydratante et la composition hydratante présente une osmolalité comparativement inférieure, pour renforcer ses propriétés hydratantes par rapport à la composition lubrifiante.

4. Un nécessaire de lubrification intime selon l'une des revendications précédentes, dans lequel les compositions sont formulées au moins substantiellement avec des constituants dérivés organiquement et de préférence au moins certains des constituants dérivés organiquement ayant été extraits sans utilisation de matières synthétiques et de préférence au moins l'un des constituants dérivés organiquement ayant été séché.

5. Un nécessaire de lubrification intime selon l'une des revendications précédentes, dans lequel les compositions contiennent soit un extrait de graine de lin soit un polysaccharide d'une autre graine, soit les deux.

6. Un nécessaire de lubrification intime selon l'une des revendications précédentes, dans lequel les compositions sont hydrophobes et de préférence la composition lubrifiante contient des constituants hydrophobes dérivés organiquement et émulsifiés.

7. Un nécessaire de lubrification intime selon l'une des revendications précédentes, dans lequel soit le pH des compositions provient de l'eau utilisée pour leur préparation soit le pH des compositions est contrôlé par addition d'agents sélectionnés parmi l'acide citrique, l'acide lactique, l'acide chlorhydrique, l'hydroxyde de sodium et l'hydroxyde de potassium, et de préférence les compositions contiennent des tampons pH.

8. Un nécessaire de lubrification intime selon l'une des revendications précédentes, dans lequel les compositions contiennent un soluté organique ou un sel inorganique pour le contrôle de l'osmolalité.

9. Un nécessaire de lubrification intime selon l'une des revendications précédentes, dans lequel la composition lubrifiante présente un pH de 5,5 à 9,0, et de préférence un pH de 7,0 à 8,5, et une osmolalité de 200 à 400 mosm et de préférence une osmolalité de 280 à 380 mosm et de préférence présente une viscosité de 10 000 à 15 000 centipoises et de préférence avec des propriétés non newtoniennes.

10. Un nécessaire de lubrification intime selon l'une des revendications précédentes, dans lequel la composition hydratante présente un pH de 3,0 à 5,5, et de préférence un pH de 3,0 à 4,5, et une osmolalité de 100 à 400 mosm et de préférence une osmolalité de 250 à 310 mosm et de préférence présente une viscosité de 12 000 à 30 000 centipoises et de préférence avec des propriétés non newtoniennes.

11. Un nécessaire de lubrification intime selon l'une des revendications précédentes, dans lequel la composition hydratante contient un mucoadhérent ou en contient plus que la première composition et contient de préférence *lactobacillus.*

12. Un nécessaire de lubrification intime selon l'une des revendications précédentes, dans lequel la composition lubrifiante contient *lonicera caprifolium* et/ou la composition hydratante contient du sorbate de potassium en tant que conservateurs.

13. Un nécessaire de lubrification intime selon l'une des revendications précédentes, comprenant une pluralité de petits récipients de la composition lubrifiante et une pluralité de récipients de la composition hydratante, les récipients étant de préférence des récipients à usage unique à long col et contenant de préférence entre 3 ml et 7 ml de leurs compositions.

14. Un nécessaire de lubrification intime selon l'une des revendications précédentes, comprenant des bandes de test d'ovulation pour déterminer la période d'ovulation.

15. Un nécessaire de lubrification intime selon l'une des revendications précédentes, comprenant des instructions d'utilisation de la composition lubrifiante pour les rapports sexuels pendant les jours allant jusqu'à l'ovulation incluse, et de la composition hydratante pendant les rapports sexuels et/ou en tant qu'hydratant utilisée après l'ovulation à tout moment jusqu'à la prochaine période fertile.
